# EUROPEAN PATENT SPECIFICATION

(11) **EP 0 858 780 B1**
(45) Date of publication and mention of the grant of the patent: **09.10.2002**
(21) Application number: 97122920.8
(22) Date of filing: 28.12.1997
(51) Int. Cl.: A61B 17/60, A61B 17/17, A61B 17/58

(54) **Orthopaedic apparatus, particularly for the surgical correction of bone deformations**
Orthopädische Vorrichtung, insbesondere zur chirurgischen Korrektur von Knockendeformationen
Appareil orthopedique, notamment pour la correction chirurgicale de deformation d'os

(30) Priority: 13.02.1997 IT VR970011
(43) Date of publication of application: 19.08.1998
(73) Proprietor: ORTHOFIX S.r.l., 37012 Bussolengo (VR) (IT)
(72) Inventor: Faccioli, Giovanni, 46040, Monzambano - Mantova (IT); Venturini, Daniele, 37065 Povegliano Veronese - Verona (IT); Ten Veldhujs, Sander, 37138 Verona (IT)
(74) Representative: Botti, Mario

(56) References cited:
- WO-A-96/05777
- WO-A-96/35386
- DE-A- 4 238 582
- US-A- 4 628 922
- US-A- 4 920 959
- US-A- 5 152 280

## Description

### Technical Field

The present invention relates to an orthopaedic apparatus, particularly for the surgical correction of bone deformations, for example of the tibia and of the femur.

### Background Art

It is known that adjustable external fixation devices are commonly utilised for correcting certain angular and longitudinal deformations of long bones. Such devices essentially comprise groups of bone screws fixed in the bone portions affected by angular defects and held by clamps which are in turn slidably mounted on longitudinal guides externally positionable at the limb to be reconstructed.

The correction normally is carried out gradually with the aid of compressor-distractor devices which act on the mobile clamps while the bone callous regenerates itself allowing a certain degree of deformation.

This methodology, however much effective, requires correction times which are rather long and does not give any immediate result for the patient.

US 4,628,922 discloses a fracture reduction apparatus comprising a first holder for holding a first set of bone fixation pins, and a second holder for holding a second set of bone fixation pins, the first and second sets of bone fixation pins being located, in use, with the site of the fracture intermediate them. Each of the holders is rotatable about a respective set of three orthogonal axes, the three axes of each set intersecting one another at a respective point located in the bone, the two respective points being spaced from one another along the longitudinal axis of the bone.

US 5,152,280 discloses a bone support device for supporting fragments of bone, in particular of fractured bones of the human body. The device includes a first member adapted to be connected to a bone fragment, a second member adapted to be connected to another bone fragment, and an adjustable articulated central body connected to the first and second members. The device allows to adjust the relative position of the two bones.

WO 96/35386 discloses a fixator for securing a first bone portion in a position relative to a second bone portion which includes a first bone screw clamp that serves as a template for drilling a hole in a first bone portion and that secures the first bone screw to the fixator, a second bone screw clamp that serves as a template for drilling a hole in the second bone portion and that secures the second bone screw to the fixator once the second bone screw is secured to the second bone portion, and a main body that is operable to secure the first bone screw clamp to the second bone screw clamp.

### Disclosure of the Invention

The present invention in accordance with one preferred aspect proposes to carry out an integral and immediate correction in the operating room of the defects obtained preliminarily by the surgeon by means of normal radiological procedures.

With this principal aim in mind, there is provided an orthopaedic apparatus, particularly for the surgical correction of angular and longitudinal defects of the bones of limbs, of the femur and tibia type, which comprises a longitudinal rod externally positionable substantially parallel to a bone for correction, a first clamp movably anchored to said rod for supporting drilling guides for screws insertable in a proximal portion of the bone, and at least one second clamp movably anchored to said rod for supporting drilling guides for screws insertable in a distal portion of the bone, said first and second clamps being orientatable and selectively blockable, before the surgical intervention, in predetermined angular positions with respect to said longitudinal rod corresponding to the angular deformations of the bone, and being repositionable with respect to said rod in correct angular positions after the osteosynthesis of the proximal and distal portions of the bone so as to eliminate the angular deformations of the bone.

### Brief Description of Drawings

The characteristics and advantages of the invention will become apparent from the description of some preferred but not exclusive embodiments of the apparatus according to the invention, illustrated for illustrative and non-limiting purposes with the help of the attached drawing sheets in which:

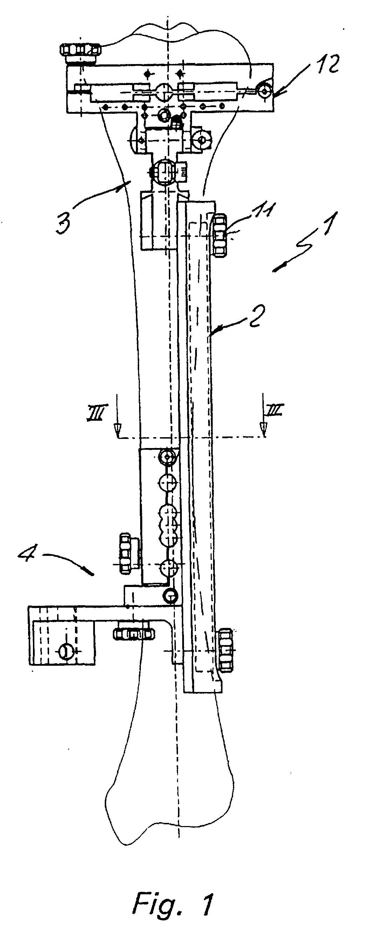

Fig. 1 illustrates a general front elevation view of the orthopaedic apparatus according to one preferred aspect of the invention applied to a bone to be corrected;

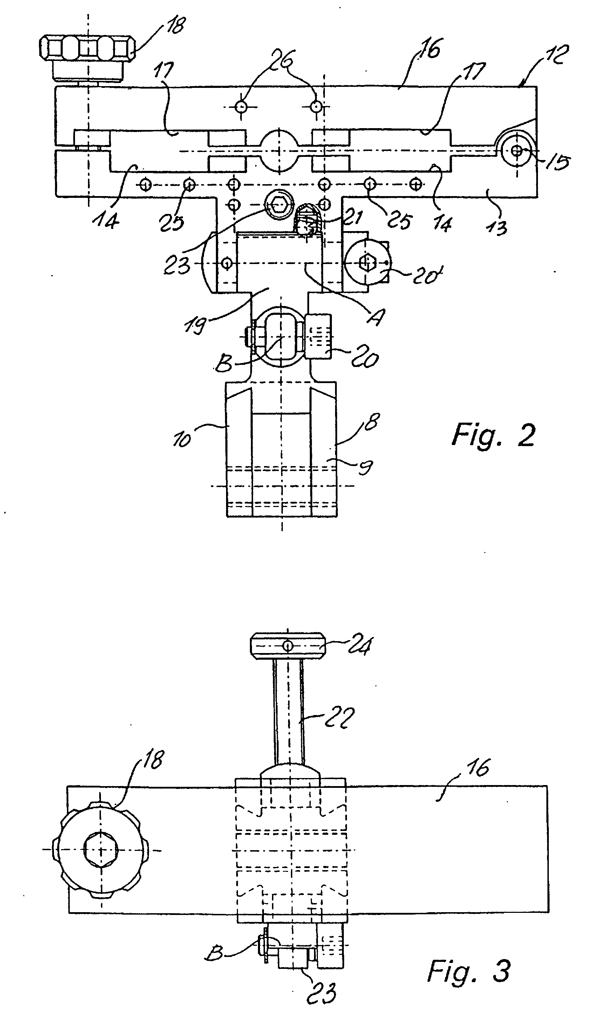

Fig. 2 illustrates a enlarged scale detail of the apparatus of Fig. 1;
Fig. 3 illustrates a top plan view of the detail of Fig. 2;

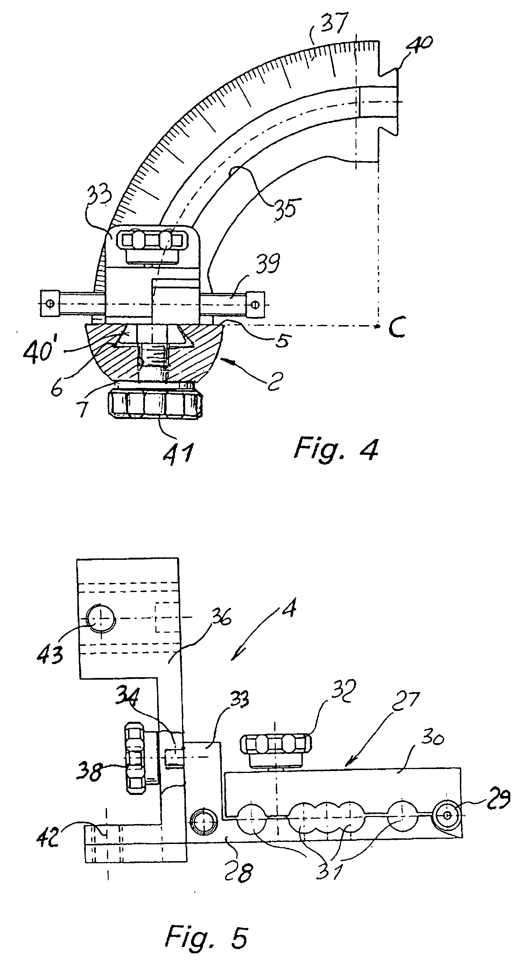

Fig. 4 illustrates a sectional view of Fig. 1 taken according to the plane III-III and shows another enlarged scale detail of the apparatus of Fig. 1;
Fig. 5 illustrates a bottom view of the detail of Fig. 4.

### Best Modes for Carrying Out the Invention

With reference to the cited figures, an orthopaedic apparatus for use in an operating room according to one preferred aspect of the invention, indicated globally by the reference numeral 1, comprises essentially a longitudinal rod 2 upon which a first end clamp 3 and a second end clamp 4, for supporting drill guides for bone screws (not shown in the drawings), are movably mounted.

The rod 2 defines a longitudinal axis Y, and has a semicircular transverse cross-section and a flat face 5 with a central longitudinal groove 6 and an elongated hole 7 which extend for nearly the entire length of the rod.

Preferably, the longitudinal groove 6 has a trapezoidal shape with the smaller side towards the flat face 5.

The first clamp 3 is substantially T-shaped with a portion 8 for connection to the rod 2 provided on its opposed faces with dovetail tenons 9, 10 countershaped with respect to the longitudinal groove 6. The clamp 3 is anchored to the rod by means of one of its tenons 9, 10 inserted in the groove 6, in proximity to one of its longitudinal ends, and is blocked thereat by means of a screw with a knob 11.

A supporting portion 12, for drilling guides of the bone screws insertable in the proximal portion of the bone, is united to the connecting portion 8, and is in turn formed by a substantially flat base plate 13 with two recesses 14 upon which a cover 16 also provided with recesses 17 is hinged at 15. The cover 16 is blocked against the base by means of a screw with a knob 18 so as to lock the drilling guides for the screws, with variable inclinations, between the recesses 14, 17.

The portion 12 is united to the portion 8 of the clamp 3 by means of an intermediate element 19 which has a first hinge axis A and a second hinge axis B, substantially perpendicular with respect to one another and with respect to the axis Y when the clamp 3 is anchored to the rod 2. In order to selectively block the angular position of the intermediate element 19 with respect to the connecting portion 8 about the axis B there is provided a first eccentric stop 20 with a hexagonal set head. In order to selectively block the angular position of the intermediate element 19 with respect to the supporting portion 12 about the axis A there is provided a second eccentric stop 20' also having a hexagonal set head.

In order to maintain the two axes A, B in preferential position there are provided sphere positioning means 21 elastically compressed by a spring against a countershaped seat formed on each axis.

There are provided distancing means constituted by a screw 22 which is screwed in a threaded hole formed in the central part of the plate 13 and having a hexagonal set head 23 and an abutment end 24 supportable at the bone. Other distancing means, for rigidly holding the bone portion in position during drilling, are constituted by Kirschner wires, not shown in the drawings, fixed in the bone and passing through calibrated holes 25 provided in the base plate 13 and calibrated holes 26 provided in the cover 16.

The second clamp 4 is essentially constituted by a second supporting portion 27, in turn formed by a base 28 upon which a cover 30 is hinged at 29. The base and the cover have facing seats 31 for holding drilling guides for the screws insertable in the distal portion of the bone, not shown in the drawings, by means of a locking action provided by a screw with a knob 32.

The base 28 has a protruding appendix 33 with an end pin 34 inserted in an arcuate groove 35 with centre C formed in a transversal circular arc element 36 having on one of its flat faces a graduated scale 37 for measuring the torsion angle of the clamp.

The centre C of the groove 35 should coincide approximately with the axis of the bone when the clamp 4 is mounted on the rod 2 and defines a third correction axis, perpendicular to the first two A, B, of the distal portion of the bone affected by torsion.

For blocking the angular position of the clamp 4, there is provided a screw with knob 38 which is screwed on the appendix 33.

There are provided distancing means constituted by a screw 39 which is screwed in a threaded hole formed in the appendix 28 and having ends adapted to be supported at the distal portion of the bone to be corrected.

At the extremities of the flat element 36 there are formed connection tenons 40, 40' which permit a right or left mounting on the rod 2 according to the requirements. The tenons 40, 40' may be inserted in the groove 6 of the rod 2 and be blocked in position by means of a screw with knob 41 which is screwed in respective threaded holes 42, 43 formed in correspondence with tenons 40, 40'.

In operation, the surgeon obtains with x-rays on multiple planes the angles of the angular defects of the proximal portion and of the torsion defects of the distal portion, and then imposes and blocks such angles on the clamps 3, 4 and mounts the latter on the rod 2. Thereafter the drilling of the bone in correspondence with the proximal and distal portions is carried out by using the drilling guides blocked on the clamps 3, 4. Then the osteotomy of the bone in correspondence with the proximal and distal portions is carried out and the clamps are brought back in correct position towards the rest position imposed by the preferential positioning means eliminating the angular defects of the bone. Finally, the clamps of the apparatus are substituted with those of an external fixator which sustains the limb for the entire period of growth of the bone callous up to complete recovery.

## Claims

1. Orthopaedic apparatus (1), particularly for the surgical correction of angular and longitudinal defects of the bones of limbs, of the femur and tibia type, comprising a longitudinal rod (2) externally positionable substantially parallel to a bone for correction, a first clamp (3) movably anchored to said rod (2) for supporting drilling guides for screws insertable in a proximal portion of the bone, and at least one second clamp (4) movably anchored to said rod (2) for supporting drilling guides for screws insertable in a distal portion of the bone, wherein the first clamp (3) has a supporting portion (12) for said drilling guides united to a first portion (8) for connection to said rod (2) and the second clamp (4) has a second supporting portion (27) for the drilling guides connected to a second portion (36) for connecting to said rod (2),
**characterized in that** said supporting portion (12) is united to said first portion (8) only by means of a first hinge axis (A) and a second hinge axis (B) substantially perpendicular with respect to one another and **in that** said second supporting portion (27) is connected to said second portion (36) only by means of circular arcuate guide means (35) with an axis (C) substantially coincident with the axis of the bone to be corrected.

2. Orthopaedic apparatus (1) according to claim 1, wherein said supporting portion (12) of said first clamp (3) is anchorable to said rod (2) such that said first and second hinging axes (A,B) are perpendicular to the longitudinal axis (Y) of the rod (2).

3. Orthopaedic apparatus (1) according to claim 1 or claim 2, wherein said first clamp (3) has preferential positioning means (21) for maintaining said supporting portion (12) in operative position substantially orthogonal with respect to said rod (2).

4. Orthopaedic apparatus (1) according to claim 3, wherein said preferential positioning means are of the sphere (21) type elastically compressed in a centering seat formed on each hinging axis. (A, B)

5. Orthopaedic apparatus (1) according to anyone of claims 1 to 4, wherein said supporting portion (12) comprises a base plate (13), a cover (16) hinged on said base (13) and a blocking knob (18) for blocking said cover against said base locking therebetween the drilling guides.

6. Orthopaedic apparatus (1) according to anyone of claims 1 to 5, wherein said first clamp (3) has adjustable distancing means (22) for positioning said supporting portion (12) with respect to the facing bone portion.

7. Orthopaedic apparatus (1) according to claim 6, wherein said distancing means comprise a screw (22) screwed in a threaded hole provided in a substantially central position of said base plate (13).

8. Orthopaedic apparatus (1) according to claim 6, wherein said distancing means comprise one or more Kirschner wires insertable in the proximal portion of the bone in calibrated holes (25, 26) formed in said base plate (13) and in said cover (16).

9. Orthopaedic apparatus (1) according to claim 1, wherein said circular arcuate guide means (35) extend in a transversal plane when said second connecting portion (35) is anchored to said rod (2).

10. Orthopaedic apparatus (1) according to claim 1, wherein said second supporting portion (27) comprises a base (28) and a cover (30) as well as screw distancing means (39) for maintaining said second claim (4) at a predetermined distance with respect to the distal portion of the bone to be corrected.

11. Orthopaedic apparatus (1) according to claim 9, wherein said rod (2) has a longitudinal seat (6) with a substantially trapezoidal transverse cross section for slidably holding first (8) and second (40, 40') connecting portions with dovetail shape of said clamps (3,4).

## Patentansprüche

1. Orthopädische Vorrichtung (1), insbesondere für die chirurgische Korrektur von Winkel- und Längsdefekten der Gliedmaßenknochen vom Femur- und Tibiatyp, umfassend einen extern im wesentlichen parallel zu einem zu korrigierenden Knochen positionierbaren Längsstab (2), eine beweglich an dem Stab (2) verankerte erste Klammer (3) zur Abstützung von Bohrfuhrungen für in einen proximalen Bereich des Knochens einbringbare Schrauben und mindestens eine beweglich an der Stange (2) verankerte zweite Klammer (4) zur Abstützung von Bohrführungen für in einen distalen Bereich des Knochens einbringbare Schrauben, wobei die erste Klammer (3) einen Abstützbereich (12) für die Bohrführungen aufweist, der mit einem ersten Bereich (8) zur Verbindung mit der Stange (2) verbunden ist und die zweite Klammer (4) einen zweiten Abstützbereich (27) für die Bohrführungen aufweist, der mit einem zweiten Bereich (36) zur Verbindung mit der Stange (2) verbunden ist,
**dadurch gekennzeichnet, daß** der Abstützbereich (12) mit dem ersten Bereich (8) lediglich mittels einer ersten Gelenkachse (A) und einer zweiten Gelenkachse (B) verbunden ist, die im wesentlichen rechtwinklig zueinander sind und daß der zweite Abstützbereich (27) mit dem zweiten Bereich (36) lediglich mittels kreisbogenförmiger Führungsmittel (35) mit einer im wesentlichen mit der Achse des zu korrigierenden Knochens koinzidenten Achse (C) verbunden ist.

2. Orthopädische Vorrichtung (1) nach Anspruch 1, wobei der Abstützbereich (12) der ersten Klammer (3) in der Weise an der Stange (2) verankerbar ist, daß die erste und zweite Gelenkachse (A, B) senkrecht zur Längsachse der (Y) der Stange (2) sind.

3. Orthopädische Vorrichtung (1) nach Anspruch 1 oder 2, wobei die erste Klammer (3) Vorzugspositionierungsmittel (21) zum Halten des Abstützbereiches (12) in Operationsposition im wesentlichen orthogonal zur Stange (2) aufweist.

4. Orthopädische Vorrichtung (1) nach Anspruch 3, wobei die Vorzugspositionierungsmittel nach Art einer Kugel (21) ausgebildet sind, die in einen auf jeder Gelenkachse (A, B) ausgebildeten Zentrierungssitz elastisch gepresst wird.

5. Orthopädische Vorrichtung (1) nach einem der Ansprüche 1 bis 4, wobei der Abstützbereich (12) eine Basisplatte (13), eine an der Basis (13) angelenkte Abdeckung (16) und einen Feststellknopf (18) zum Feststellen der Abdeckung gegenüber der Basis und Festsetzen der Bohrführungen dazwischen enthält.

6. Orthopädische Vorrichtung (1) nach einem der Ansprüche 1 bis 5, wobei die erste Klammer (3) einstellbare Abstandsmittel (22) für die Positionierung des Abstützbereiches (12) gegenüber dem zugewandten Knochenbereich aufweist.

7. Orthopädische Vorrichtung (1) nach Anspruch 6, wobei die Abstandsmittel eine Schraube (22) umfassen, die in eine in einer im wesentlichen zentralen Position der Basisplatte (13) vorgesehene Gewindebohrung eingeschraubt ist.

8. Orthopädische Vorrichtung (1) nach Anspruch 6, wobei die Abstandsmittel einen oder mehrere Kirschner-Drähte umfasst, die im proximalen Bereich des Knochens in kalibrierte, in der Basisplatte (13) und der Abdeckung (16) eingebrachte Bohrungen (25, 26) einsetzbar sind.

9. Orthopädische Vorrichtung (1) nach Anspruch 1, wobei die kreisbogenförmigen Führungsmittel (35) sich in einer transversalen Ebene erstrecken, wenn der zweite Verbindungsbereich (36) an der Stange (2) verankert ist.

10. Orthopädische Vorrichtung (1) nach Anspruch 1, wobei der zweite Abstützbereich (27) eine Basis (28) und eine Abdeckung (30) sowie Schraubenabstandsmittel (39) zum Halten der zweiten Klammer (4) in einem vorbestimmten Abstand zum distalen Bereich des zu korrigierenden Knochens umfasst.

11. Orthopädische Vorrichtung (1) nach Anspruch 9, wobei die Stange (2) einen Längssitz (6) mit einem im wesentlichen trapezförmigen Querschnitt zum verschiebbaren Halten von ersten (8) und zweiten (40, 40') Verbindungsabschnitten der Klammern (3, 4) mit Schwalbenschwanzform aufweist.

## Revendications

1. Appareil orthopédique (1), en particulier pour la correction chirurgicale de défauts angulaires et longitudinaux d'os de membres, du type fémur et tibia, comprenant une tige longitudinale (2), susceptible d'être positionnée de façon externe sensiblement parallèlement à un os pour sa correction, une première pince (3) attachée de manière mobile à ladite tige (2) pour supporter des guides de perçage pour des vis susceptibles d'être insérées dans une partie proximale de l'os et au moins une deuxième pince (4) attachée de manière mobile à ladite tige (2) pour supporter des guides de perçage pour des vis susceptibles d'être insérées dans une partie distale de l'os, dans lequel la première pince (3) possède une partie de support (12) pour lesdits guides de perçage, jointe à une première partie (8) pour être connectée à ladite tige (2), et la deuxième pince (4) possède une deuxième partie de support (27) pour les guides de perçage, jointe à une deuxième partie (36) pour être connectée à ladite tige (2), **caractérisé en ce que** ladite partie de support (12) est jointe à ladite première partie (8) seulement au moyen d'un premier axe d'articulation (A) et d'un deuxième axe d'articulation (B), sensiblement perpendiculaires l'un par rapport à l'autre, et **en ce que** ladite deuxième partie de support (27) est connectée à ladite deuxième partie (36) seulement au moyen de moyens de guidage en arc de cercle (35) avec un axe (C) coïncidant sensiblement avec l'axe de l'os devant être corrigé.

2. Appareil orthopédique (1) selon la revendication 1, dans lequel ladite partie de support (12) de ladite pince (3) est susceptible d'être attachée à ladite tige (2) de manière à ce que lesdits premier et deuxième axes d'articulation (A, B) soient perpendiculaires à l'axe longitudinal (Y) de la tige (2).

3. Appareil orthopédique (1) selon la revendication 1 ou 2, dans lequel ladite première pince (3) possède des moyens de positionnement préférentiels (21) pour maintenir ladite partie de support (12) en une position opérationnelle sensiblement orthogonale par rapport à ladite tige (2).

4. Appareil orthopédique (1) selon la revendication 3, dans lequel lesdits moyens de positionnement préférentiels sont de type sphère (21), comprimés élastiquement dans un siège de centrage ménagé sur chaque axe d'articulation (A, B).

5. Appareil orthopédique (1) selon l'une quelconque des revendications 1 à 4, dans lequel ladite partie de support (12) comprend une plaque de base (13), un couvercle (16) articulée sur ladite base (13) et un bouton de blocage (18) pour bloquer ledit couvercle contre la base en verrouillant entre eux les guides de perçage.

6. Appareil orthopédique (1) selon l'une quelconque des revendications 1 à 5, dans lequel ladite première pince (3) possède des moyens ajustables de distanciation (22) pour positionner ladite partie de support (12) par rapport à la partie faisant face à l'os.

7. Appareil orthopédique (1) selon la revendication 6, dans lequel lesdits moyens de distanciation comprennent une vis (22) vissée dans un trou fileté prévu en une position sensiblement centrale de ladite plaque de base (13).

8. Appareil orthopédique (1) selon la revendication 6, dans lequel lesdits moyens de distanciation comprennent un ou plusieurs fils de Kirschner susceptibles d'être insérés dans la partie proximale de l'os dans des trous calibrés (25, 26) ménagés dans ladite plaque de base (13) et ladite couverture (16).

9. Appareil orthopédique (1) selon la revendication 1, dans lequel lesdits moyens de guidage en arc de cercle (35) s'étendent dans un plan transversal lorsque ladite deuxième partie de connexion (36) est attachée à ladite tige (2).

10. Appareil orthopédique (1) selon la revendication 1, dans lequel lesdits deuxièmes moyens de support (27) comprennent une base (28) et une couverture (30), de même que des moyens de distanciation à vis (39), pour maintenir ladite deuxième pince (4) à une distance prédéterminée par rapport à la partie distale de l'os devant être corrigé.

11. Appareil orthopédique (1) selon la revendication 9, dans lequel ladite tige (2) possède un siège longitudinal (6) avec une section transversale sensiblement trapézoïdale pour maintenir de façon coulissante des première et deuxième parties de connexion (40, 40') avec une forme de queue d'aronde desdites pinces (3, 4).
